(19) Europäisches Patentamt
European Patent Office
Office européen des brevets

(11) Publication number: **0 495 047 B1**

# EUROPEAN PATENT SPECIFICATION

(45) Date of publication of patent specification: **29.11.95**

(51) Int. Cl.⁶: **A61K 38/42**, A61K 35/18

(21) Application number: **91913972.5**

(22) Date of filing: **24.07.91**

(86) International application number:
**PCT/US91/05219**

(87) International publication number:
**WO 92/02239 (20.02.92 92/05)**

(54) **STABLE HEMOGLOBIN BASED COMPOSITION AND METHOD TO STORE SAME.**

(30) Priority: **06.08.90 US 562786**

(43) Date of publication of application:
**22.07.92 Bulletin 92/30**

(45) Publication of the grant of the patent:
**29.11.95 Bulletin 95/48**

(84) Designated Contracting States:
**AT BE CH DE DK FR GB IT LI LU NL**

(56) References cited:
**EP-A- 216 639**

**BIOLOGICAL ABSTRACTS, vol. 87, no. 5, 1989; S.M. CHRISTENSEN, pp. 143-154, no. 44568**

**CHEMICAL ABSTRACTS, vol. 87, no. 117, 24 October 1977, Columbus, OH (US); M. KRAM-LOVA, p. 437, no. 131414g**

(73) Proprietor: **BAXTER INTERNATIONAL INC.**
**One Baxter Parkway**
**Deerfield, IL 60015 (US)**

(72) Inventor: **KANDLER, Richard, L.**
**2720 Myang**
**McHenry, IL 60050 (US)**
Inventor: **SPICUSSA, John, C.**
**650 Central Avenue**
**Highland Park, IL 60035 (US)**

(74) Representative: **MacGregor, Gordon et al**
**ERIC POTTER CLARKSON**
**St. Mary's Court**
**St. Mary's Gate**
**Nottingham, NG1 1LE (GB)**

CHEMICAL ABSTRACTS, vol. 111, no. 21, 20 November 1989, Columbus, OH (US); A. MANSOURI, p. 344, no. 190140b

CHEMICAL ABSTRACTS, vol. 108, no.11, 14 March 1988, Columbus, OH (US); T. SAITO, p. 457, no. 92302z

CHEMICAL ABSTRACTS, vol. 107, no. 26, 28 December 1987, Columbus, OH (US); D. ZYG-MUNT, p. 369, no. 242487j

CHEMICAL ABSTRACTS, vol. 85, no. 24, 13 December 1976, Columbus, OH (US); P. LABRUDE, p. 415, no. 18011u

CHEMICAL ABSTRACTS, vol. 95, no. 14, 05 October 1981, Columbus, OH (US); L.R. SEH-GAL, p. 358, no. 121024f

**Description**

This invention relates to a stable hemoglobin-based oxygen-carrying composition and methods to store or process same, said hemoglobin composition including hemoglobin, modified hemoglobin and encapsulated hemoglobin.

Description of Related Art

Although human whole blood and packed red cell preparations have long been used to restore body oxygen-carrying function, hemoglobin-based compositions are now seen to offer several advantages over these currently standard hemotherapies. Patient safety would be enhanced by eliminating untoward reactions to blood group antigens, improving the maintenance of systemic acid-base and electrolyte balance, and avoiding exposure to blood contaminated with diseases such as AIDS and hepatitis. The high costs of testing blood for blood group antigens and disease factors would also be avoided. Further, hemoglobin-based oxygen-carrying substitutes are expected to have prolonged stability during storage so a more stable supply should be realized.

While a number of parameters may be measured to characterize hemoglobin solution stability, such as oxygen carrying capacity and ionic composition, the most important of these is the oxidation state of hemoglobin iron. The iron atom in each of the heme prosthetic groups of the hemoglobin molecule is the site of oxygen binding and release. In order to maintain this reversible oxygen binding capability, the heme iron must be in the physiological $Fe^{2+}$ state. When a solution of hemoglobin is stored for a long period of time, the iron tends to oxidize to the $Fe^{3+}$ state, giving the methemoglobin form which does not reversibly bind oxygen and is therefore physiologically ineffective. (H.F. Bunn et al. Hemoglobin: Molecular Genetic and Clinical Aspects 640 (1986)).

One current strategy to prevent the above described hemoglobin autoxidation reation is the addition of anti-oxidants (Stratton, L.P. Hemoglobin 12(4):353-368(1988)) such as NADH (Kajita, A. et al., Biochem. Biophys. Res. Comm. 39:1199(1970)). However, in hemoglobin solutions some anti-oxidants can become pro-oxidants (ibid. Stratton et al.). Further, the addition of a component to an intravenous solution product complicates manufacture and raises issues of the toxicity of the new component which can require costly testing during development.

The active reduction of methemoglobin in solution can be accomplished by electrochemical means (P. Scheller, Mechanism of Cathodic Reduction of Hemoproteins, 60 Studia Biophysica 137 (Berlin) (1976)), the use of reductive enzyme systems (Hayashi. A. et al. Biochem. Biophys. Acta 310:309(1973)) or the addition of chemical reducing agents such as ascorbate (Tomoda, A. et al., J. Biol. Chem. 253 (20):7415-7419 (1978)). These systems all require chemical additives and suffer from the same limitations mentioned above for anti-oxidants.

A viable hemoglobin based oxygen-carrying composition should contain less than 50% and preferably less than 15% methemoglobin over the shelf life. The rate of hemoglobin auto-oxidation is, of course, dependent on temperature. Thus,at room temperature (25°C) heme iron oxidation can exceed 30% per day (see Devenuto. F., infra at 946). Consequently, hemoglobin based oxygen carrying compositions are typically stored in the frozen state to obtain the targeted shelf-life.

The extent of hemoglobin protein purity is an important consideration in any discussion of hemoglobin stability in solution. Hemoglobin heme iron is continuously oxidized in vivo and the red cell contains enzyme systems that directly reduce methemoglobin to restore hemoglobin function. (Hultquist D.E. et al., The Methemoglobin Reduction System of Erythrocytes, in The International Conference on Red Cell Metabolism and Function, 297-301; Univ. of Michigan (1974)). Paul H.B. et al, Preparation of Hemoglobin Solutions for Intraveneous Infusions, 455, 463 (1947). Other red cell enzymes eliminate activated oxygen products, such as superoxide and hydrogen peroxide, that can also oxidize heme iron. (Watkins J.A. et al., Biochem. Biophys. Res. Comm. 132(2):742-748 (1985)). It is not surprising therefore that less pure preparations of hemoglobin exhibit increased oxidation stability, particularly when the native methemoglobin reductase substrates, such as NADH, are added to the preparation (Stratton, L.P. et al, Hemoglobin 12(4)-:353-368 (1988)). Despite this oxidative protection in impure preparations, contaminating proteins can cause immunogenic reactions in patients or may possess innate toxic properties when free in the circulation. Thus, a system that could provide oxidation stability and/or reduce methemoglobin in purified hemoglobin preparations, over a broad temperature range, would possess a substantial advantage.

EP 0 495 047 B1

## Summary of the Invention

This invention relates to methods to obtain a set of conditions as delineated below, that allow the expression of a hemoglobin autoreduction reaction that spontaneously converts methemoglobin to deoxyhemoglobin in purified hemoglobin preparations This autoreduction reaction is accelerated at higher temperatures, and as long as the proper conditions are maintained as delineated below, the system provides oxidation stability at room temperature.

The present invention provides a method of preparing a hemoglobin based composition having methemoglobin in an amount not greater than 15% of total hemoglobin, wherein the composition may function as an oxygen carrying solution upon administration to a patient,

the method comprising removing oxygen from an oxygen impermeable container, adding a purified substantially deoxygenated hemoglobin solution to the container and storing the container at a temperature of 5°C to 45°C for a sufficient time to permit autoreduction of methemoglobin.

The invention also provides a method of reducing methemoglobin in a solution of substantially deoxygenated and purified methemoglobin, the method comprising removing oxygen from an oxygen impermeable container, adding the deoxygenated and purified hemoglobin solution to the container, and storing the container at a temperature of 5°C to 45°C for a sufficient time for autoreduction of methemoglobin in the solution to occur, such that the resultant solution contains methemoglobin in an amount not greater than 15% of total hemoglobin, so that the solution may function as an oxygen carrying solution upon administration to a patient.

The invention also provides a method for packaging a hemoglobin solution, comprising purging an oxygen permeable container with nitrogen,

preparing a thoroughly deoxygenated, purified hemoglobin solution,

filling said purged container with an aliquot of said thoroughly deoxygenated, purified hemoglobin solution, and

sealing the container,

whereby methemoglobin in the solution is autoreducible to a level not greater than 15% of the total hemoglobin when the sealed container is stored at a temperature of 5° to 45°C for a sufficient amount of time to permit autoreduction to said level such that the solution is usable as an oxygen carrying solution upon administration to a patient.

The purified hemoglobin based composition includes hemoglobin, modified hemoglobin and/or encapsulated hemoglobin preparations that have been purified. Purification can be according to any known standard method such as heat treatment. (See Estep, U.S. Patent Nos. 4,861,867 and 4,831,012 (incorporated by reference)). To effectively restore body oxygen-carrying function, it is presently believed that a stable hemoglobin based composition preferably has less than 15%, of the composition in the methemoglobin form.

It is further an object of this invention to stop methemoglobin production and in fact cause $Fe^{3+}$ methemoglobin to convert to reduced $Fe^{2+}$ hemoglobin in said composition.

It is further an object of this invention to store a purified hemoglobin based composition in a system that actively scavenges oxygen, thus allowing the autoreduction reaction to predominate over autoxidation.

It is still another object of this invention to provide a system of oxygen removal that does rot require pretreatment or entry into the storage system. The hemoglobin solution, which may contain an amount of methemoglobin, is stored in a container that is substantially impermeable to oxygen. In this substantially impermeable oxygen environment the hemoglobin autoxidation reaction will consume any oxygen in solution, deoxygenate the remaining $Fe^{2+}$ hemoglobin and allow autoreduction of any methemoglobin to proceed.

It is still another object of this invention to store a purified hemoglobin based composition in the oxygenated form and allow deoxygenation in the container to permit autoreduction.

## Brief Description of Drawings

For a fuller understanding of the nature and objects of the invention, reference should be made to the following detailed description taken in connection with the accompanying description forming a part of this specification.

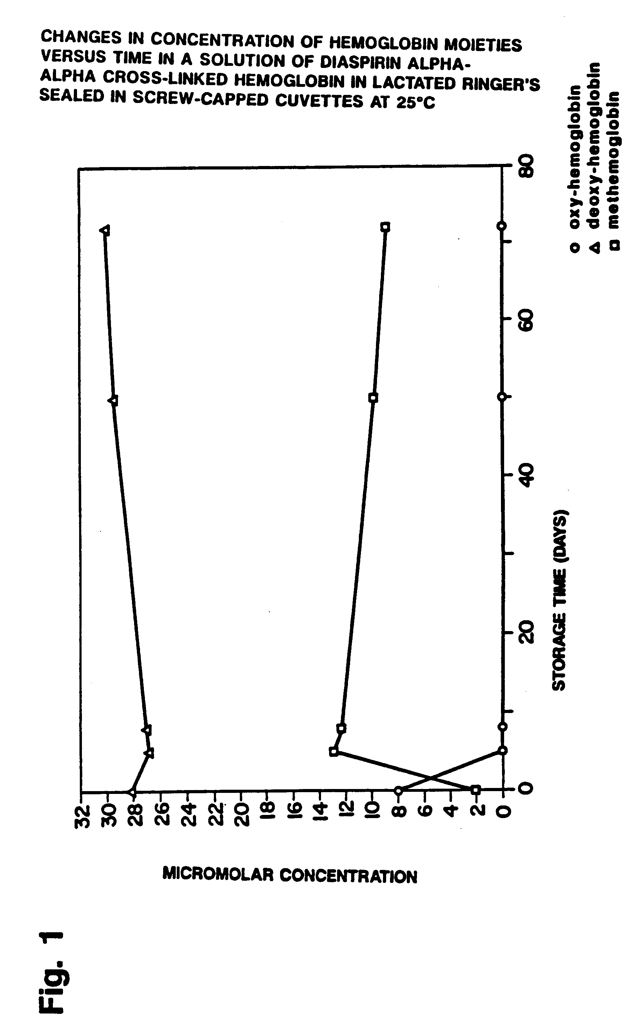

FIG. 1 shows the changes in concentration of hemoglobin moieties versus time in a solution of diaspirin alpha-alpha cross-linked hemoglobin in lactated Ringer's (total diaspirin cross-linked hemoglobin concentration of approximately 39$\mu$M) that was sealed in a screw-topped cuvette (no head-space) and kept at 25°C.

4

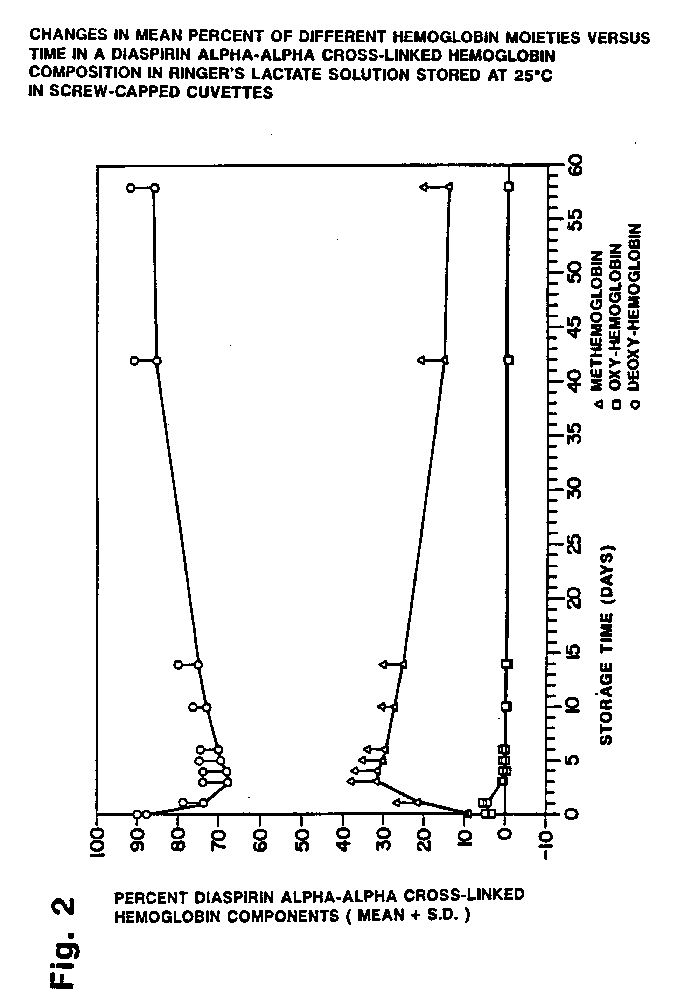

FIG. 2 shows changes in mean percent hemoglobin moieties versus time in sealed screw-capped cuvettes (N = 4) that were filled completely with a deoxygenated solution of diaspirin cross-linked hemoglobin in lactated Ringer's (total diaspirin cross-linked hemoglobin concentration of approximately $38\mu$) and kept at 25°C.

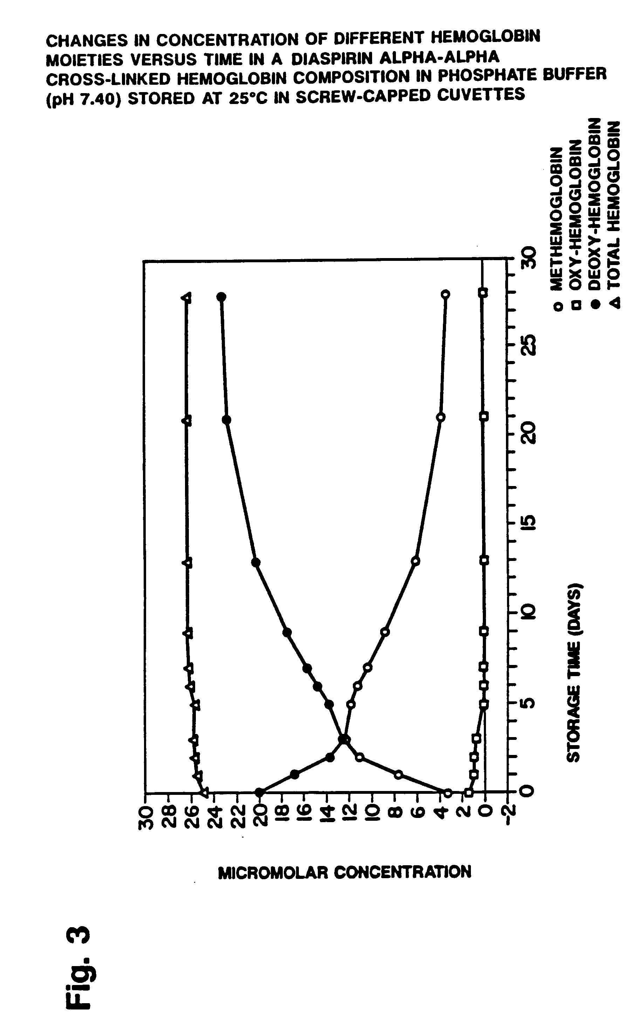

FIG. 3 shows the changes in hemoglobin moities and total hemoglobin versus time in a deoxygenated solution of diaspirin cross-linked hemoglobin in 10mM phosphate buffer that was sealed in a screw-capped cuvette (no head space) and kept at 25°C.

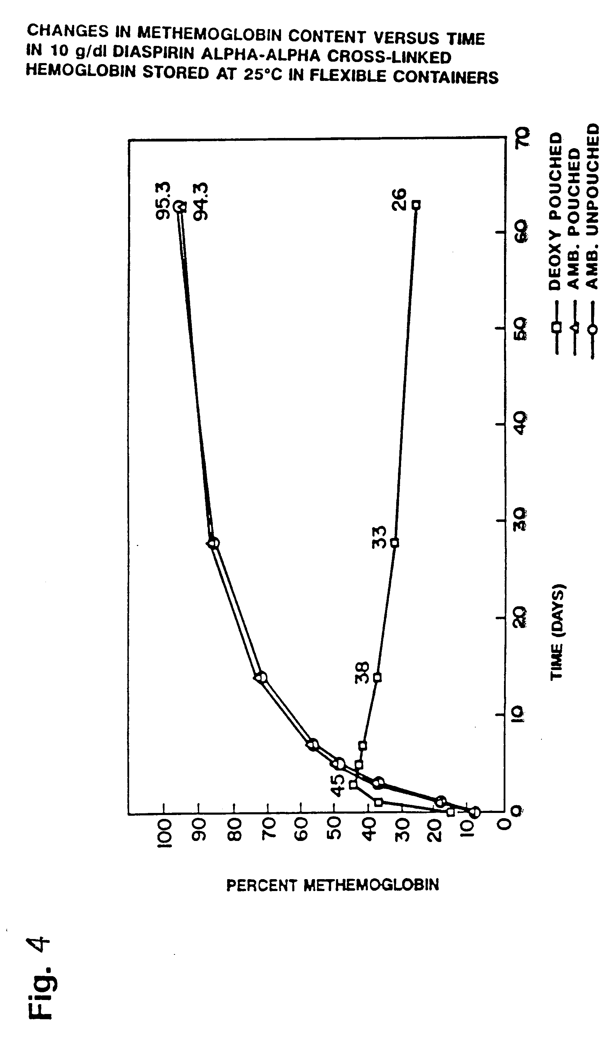

FIG. 4 shows the changes in percent methemoglobin versus time in a solution of 10g/dl diaspirin cross-linked hemoglobin in lactated Ringer's. The control group units were not deoxygenated and were either sealed in foil pouches (Ambient Pouched) or not (Ambient Not Pouched). All units were kept at 25°C.

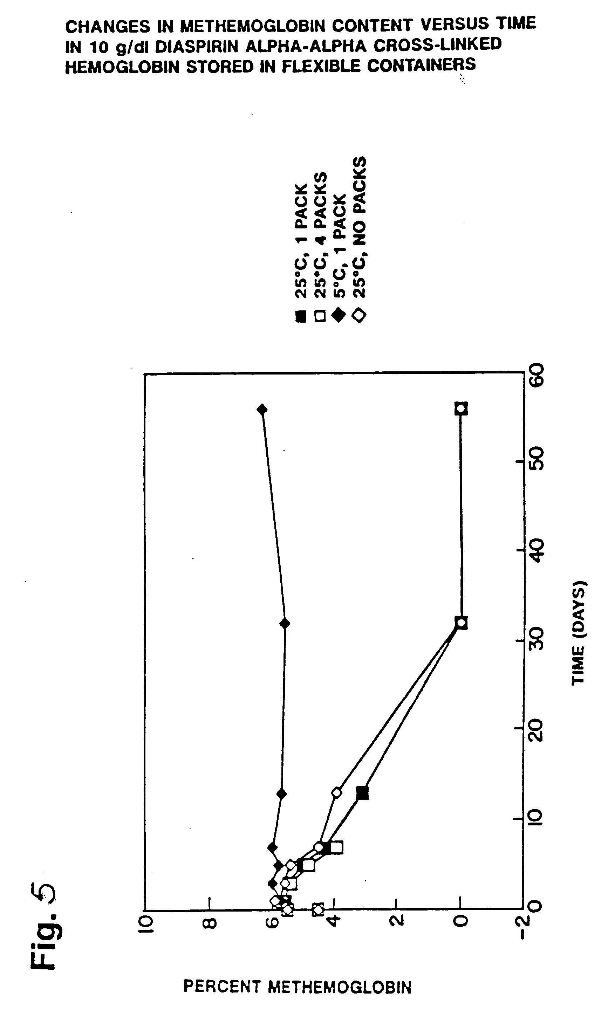

FIG. 5 shows the changes in percent methemoglobin versus time in a deoxygenated solution of 10g/dl diaspirin cross-linked hemoglobin in lactated Ringer's. Units in the three test groups units were stored at 5°C with one 100ml capacity oxygen absorbing packet in the foil pouch, 25°C with one 100ml packet or 25°C with four 100ml packets. Control group units were stored at 25°C with no oxygen absorbing packets in the foil pouch.

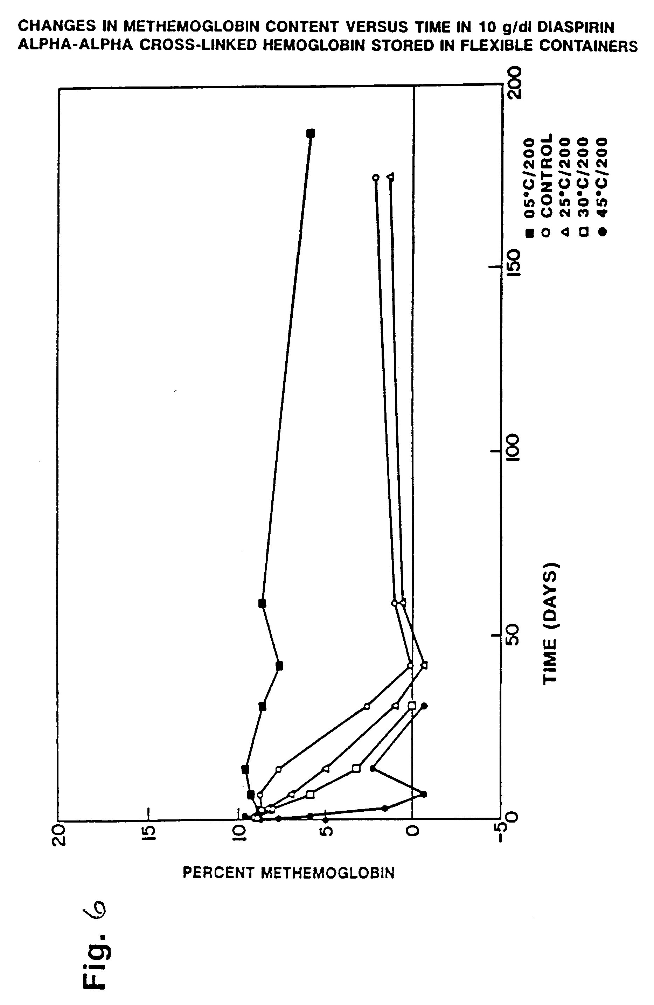

FIG. 6 shows the changes in percent methemoglobin versus time in a deoxygenated solution of 10 g/dl diaspirin cross-linked hemoglobin in lactated Ringer's. The packaging of the solution was carried out as in Example 6 above. Test group units contained a 200 ml capacity oxygen absorbing packet in the foil pouch and were stored at 5°C, 25°C, 30°C, or 45°C. Control group unit storage at 25°C was without oxygen packets.

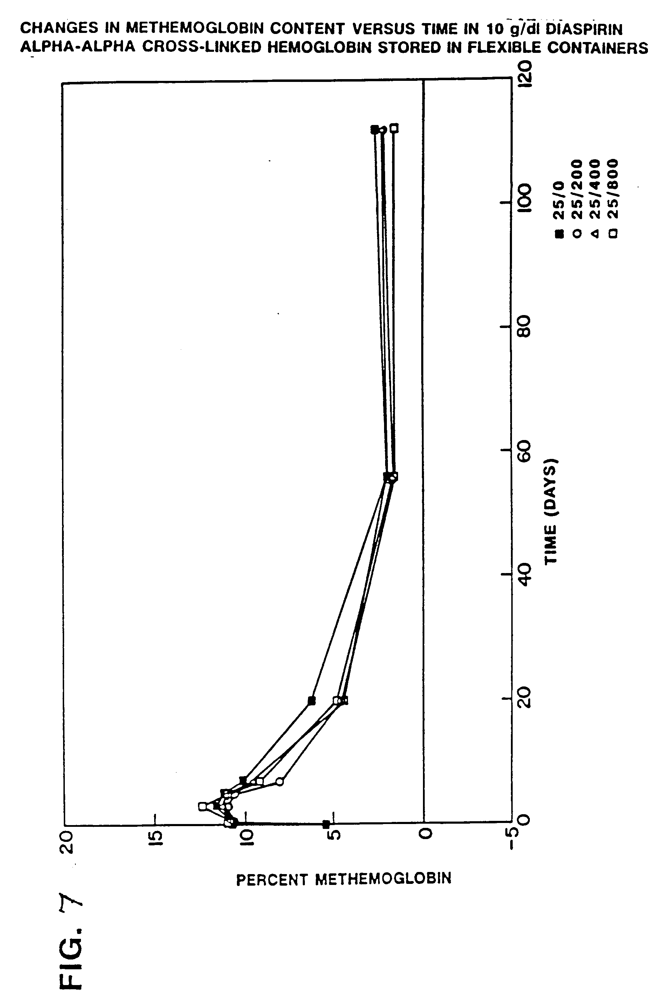

FIG. 7 shows the changes in percent methemoglobin versus tine in a deoxygenated solution of 10 g/dl diaspirin cross-linked hemoglobin in lactated Ringer's. Test group units contained 200, 400 or 800 ml of oxygen absorbing capacity while the control group units contained no packets.

Detailed Description of the Invention - Best Mode

We have observed that in deoxygenated solutions of purified hemoglobin, oxidized $Fe^{3+}$ methemoglobin heme iron will spontaneously reduce to the physiologically useful $Fe^{2+}$ form. Based on this discovery we have developed a packaging and storage system that allows the expression of the autoreduction reaction.

The components necessary to perform the packaging operation are sealed in an isolator (glove chamber). These components would include, for example, plastic bag primary containers for the hemoglobin solution, outer wrap secondary containers that have very low oxygen permeability, pumps, clamps, tubing assemblies, a solution reservoir, and oxygen monitors. The isolator interior is sterilized with a vaporized sterilant such as $H_2O_2$ and then cleared of residual sterilant with low-oxygen grade nitrogen. Oxygen is then rigorously removed from the isolator by carefully purging the isolator interior and all components within it with nitrogen. Preferably, at this stage ultra-pure grade nitrogen would be used with in-line oxygen traps to attain very low oxygen levels.

In the now low oxygen environment of the isolator, the plastic primary containers are filled with aliquots of a thoroughly deoxygenated hemoglobin solution and then sealed in foil pouch secondary containers. When this operation is completed the isolator is opened and the containers are placed in storage.

Although autoreduction has been observed to take place during the deoxygenation of the hemoglobin solution, and can be taken advantage of in this way, the reaction proceeds readily during storage in a substantially oxygen impermeable package and requires no further attention.

After hemoglobin solutions are prepared and packed as described above, there is typically a period of two to five days before the onset of autoreduction. During this time residual oxygen is scavenged from the solution by the oxidation reaction. Consequently, this oxygen scavenging phase is characterized by either an increase in methemoglobin or, if deoxygenation of the solution and packaging components was rigorous enough, a time during which methemoglobin remains at the initial level. The latter case occurs when oxygen has been reduced to a level at which the rates of autoxidation and autoreduction are equal. The duration of this oxygen scavenging phase is temperature dependent, ranging from less then one day at 45°C to months at 5°C.

After the oxygen scavenging phase the methemoglobin level begins to decrease and this trend continues in an essentially linear manner to a minimum, generally less than 5% methemoglobin, that is maintained as long as oxygen is substantially excluded.

The acceptable temperature range for reducing methemoglobin in a purified hemoglobin composition during storage is between 5°C, where the autoreduction reaction is very slow, to less than 45°C above which other degradant reactions can limit shelf-life. Room temperature oxidation stability is achieved with this system. Among the examples below, ongoing experiments are described in which, subsequent to the oxygen-scavenging phase, methemoglobin has been maintained at less than three percent for 231 days at 25°C.

It should be noted that once methemoglobin concentration is sufficiently low to allow said hemoglobin solution to effectively function as an oxygen carrying solution the solution can be stored at from between -270°C to 45°C.

Example 1

In this experiment a dilute solution of alpha-alpha cross-linked hemoglobin was sealed in screw-capped cuvettes and kept at 25°C. With a dilute solution (approximately 39 $\mu$M diaspirin cross-linked hemoglobin) in a cuvette it was possible to noninvasively observe changes in the concentrations of oxy-, deoxy-, met- and total hemoglobin during storage using a standard three-wavelength spectrophotometric method taking measurements at 560, 576 and 630nm.

All manipulations to prepare samples were carried out in a glove-bag continuously purged with ultra-pure nitrogen. One liter of a dilute solution of purified diaspirin cross-linked hemoglobin in lactated Ringer's solution was deoxygenated by recirculation through a membrane oxygenator charged with ultra-pure nitrogen. The deoxygenated diaspirin cross-linked hemoglobin solution was sealed in screw-capped cuvettes which were stored at 25°C.

Despite the efforts to exclude oxygen, the sample initially contained 20% oxy diaspirin cross-linked hemoglobin, 5.5% met- and 73.7% deoxy diaspirin cross-linked hemoglobin. At five days no detectable oxy diaspirin cross-linked hemoglobin was present and the methemoglobin had risen to 32.5%. The balance was in the deoxy state and total hemoglobin was unchanged (Table 1). These results illustrate the deoxygenation of a hemoglobin solution by the oxidation reaction during the oxygen scavenging phase.

In subsequent measurements at days 8, 50 and 72 the methemoglobin steadily decreased. See FIG. 1. It is important to observe the actual concentration changes of all three hemoglobin forms and total hemoglobin during the autoreduction rather than percent changes. If methemoglobin were precipitating the percent of that component and the total hemoglobin concentration would fall. The concentration data in Table 1, show the essentially quantitative autoreduction of methemoglobin to deoxyhemoglobin while the total concentration of diaspirin cross-linked hemoglobin remained constant.

TABLE 1

| Changes in Percent Content and Micromolar Concentrations of Hemoglobin Moieties in a Solution of Diaspirin Cross-Linked Hemoglobin in Lactated Ringer's Sealed in a Cuvette at 25°C | | | | | | |
|---|---|---|---|---|---|---|
| | PERCENT OF TOTAL DIASPIRIN CROSS-LINKED HEMOGLOBIN | | | MICROMOLAR CONCENTRATION | | |
| DAY | met | oxy | deoxy | met | oxy | deoxy |
| 0 | 5.5 | 20.8 | 73.7 | 2.1 | 8.0 | 28.2 |
| 5 | 32.5 | ND | 67.8 | 12.9 | ND | 26.9 |
| 8 | 31.5 | ND | 68.9 | 12.3 | ND | 27.0 |
| 50 | 25.1 | ND | 75.6 | 9.8 | ND | 29.4 |
| 72 | 23.0 | ND | 77.9 | 8.9 | ND | 30.1 |
| ND = not detected. | | | | | | |

Example 2

In this experiment, efforts were taken to ensure that the samples were stored in sufficiently oxygen impermeable containers to observe autoreduction and obtain an estimate of experimental error in these studies. A dilute solution of diaspirin cross-linked hemoglobin in lactated Ringer's was prepared and deoxygenated as described in Example 1.

Although an increase in the oxyhemoglobin derivative on day one (FIG. 2, Table 2) indicated oxygen leaks, the results obtained in Example 1 with respect to the quantitative conversion of $Fe^{3+}$ methemoglobin to $Fe^{2+}$ deoxyhemoglobin were reproduced in the four samples prepared within a reasonable degree of precision (see + S.D. bars in FIG. 2). The mean values of the percent content and micromolar

**EP 0 495 047 B1**

concentration of the hemoglobin moieties in the sealed cuvettes (N = 4) are given in Table 2.

7

TABLE 2

| Changes in Mean Percent of Total Diaspirin Cross-Linked Hemoglobin and Micromolar Concentration of Hemoglobin Moieties at 25 °C in Screw-Capped Cuvettes Containing a Solution of Diaspirin Cross-Linked Hemoglobin in Lactated Ringer's | | | | | | |
|---|---|---|---|---|---|---|
| | PERCENT OF TOTAL DIASPIRIN CROSS-LINKED HEMOGLOBIN | | | MICROMOLAR CONCENTRATION | | |
| DAY | met | oxy | deoxy | met | oxy | deoxy |
| 0 | 8.8 | 3.3 | 87.9 | 2.4 | 0.9 | 23.6 |
| 1 | 21.6 | 4.5 | 73.9 | 6.0 | 1.3 | 20.4 |
| 3 | 31.8 | 0.5 | 67.7 | 8.9 | 0.1 | 18.9 |
| 4 | 31.8 | 0.2 | 68.2 | 8.8 | 0.1 | 19.1 |
| 5 | 30.3 | 0.1 | 69.6 | 8.4 | 0.0 | 19.3 |
| 6 | 29.8 | 0.1 | 70.1 | 8.4 | 0.0 | 19.6 |
| 10 | 27.5 | ND | 72.8 | 7.8 | ND | 20.7 |
| 14 | 25.5 | ND | 74.9 | 7.2 | ND | 21.1 |
| 42 | 15.4 | ND | 85.2 | 4.4 | ND | 24.0 |
| 58 | 14.5 | ND | 86.1 | 4.2 | ND | 24.5 |
| ND = not detected. | | | | | | |

Example 3

In the previous examples of the autoreduction reaction in oxygen impermeable systems the diaspirin cross-linked hemoglobin was in a lactated Ringer's medium. To ensure that the observed autoreduction effect was not due to a component in the lactated Ringer's, diaspirin cross-linked hemoglobin was exchanged into phosphate ($PO_4$) buffer. Twenty ml of 10 g/dl diaspirin cross-linked hemoglobin in lactated Ringer's was diluted to one liter with 10mM $PO_4$; pH = 7.40. This fifty-fold dilution was a 98% change of medium. This diluted solution was diafiltered vs. five liters of the same phosphate buffer to effect a further theoretical 99% change in composition of the buffer to phosphate buffer.

This solution of diaspirin cross-linked hemoglobin in phosphate was deoxygenated and a number of screw-capped cuvettes were filled, and sealed as described in Example 1. The characteristic pattern of oxidation followed by autoreduction was observed at faster oxidation and reduction rates relative to Examples 1 and 2. The data from one representative cuvette are given in Table 3 and FIG. 3. The methemoglobin level fell 36.5% (9.3$\mu$M) in twenty-five days.

TABLE 3

| Changes in Percent Content and Micromolar Concentration of Hemoglobin Moieties at 25°C in Screw-Capped Cuvette Containing Diaspirin Cross-Linked Hemoglobin in Phosphate Buffer | | | | | | | |
|---|---|---|---|---|---|---|---|
| | PERCENT OF TOTAL DIASPIRIN CROSS-LINKED HEMOGLOBIN | | | MICROMOLAR CONCENTRATION | | | |
| DAY | met | oxy | deoxy | met | oxy | deoxy | Total Deoxy Hemoglobin |
| 0 | 13.5 | 5.7 | 80.8 | 3.4 | 1.4 | 20.1 | 24.9 |
| 1 | 30.0 | 4.0 | 65.9 | 7.7 | 1.0 | 16.8 | 25.5 |
| 2 | 42.9 | 3.8 | 53.3 | 11.0 | 1.0 | 13.7 | 25.7 |
| 3 | 48.6 | 3.0 | 48.4 | 12.5 | 0.8 | 12.5 | 25.8 |
| 5 | 45.9 | 0.5 | 53.5 | 11.8 | 0.1 | 13.8 | 25.7 |
| 6 | 43.2 | 0.3 | 56.4 | 11.3 | 0.1 | 14.7 | 26.1 |
| 7 | 39.7 | 0.3 | 60.0 | 10.4 | 0.1 | 15.7 | 26.2 |
| 9 | 33.6 | 0.0 | 66.4 | 8.8 | ND | 17.4 | 26.2 |
| 13 | 23.1 | ND | 77.1 | 6.1 | ND | 20.2 | 26.3 |
| 21 | 14.1 | ND | 86.5 | 3.7 | ND | 22.6 | 26.3 |
| 28 | 12.1 | ND | 88.2 | 3.2 | ND | 23.0 | 26.3 |
| ND = not detected. | | | | | | | |

Example 4

The previous examples demonstrate that spontaneous autoreduction occurs with dilute hemoglobin solutions (approximately 40 $\mu$M; 0.29 g/dl of hemoglobin) stored in oxygen impermeable containers. In this experiment we assessed when autoreduction would occur using a concentrated hemoglobin solution (10 g/dl).

All the experimental components necessary to deoxygenate and package the diaspirin cross-linked hemoglobin solution were assembled in a gloved isolator chamber. The isolator was sealed and purged with low-oxygen grade nitrogen to 0.017 ppm oxygen. The 10g/dl diaspirin cross-linked hemoglobin solution was deoxygenated to two percent oxygen. The test group flexible containers (150 ml internal volume) were filled with approximately 50 ml of deoxygenated diaspirin cross-linked hemoglobin each and then sealed in substantially oxygen impermeable foil pouches.

Two groups of control units were filled under ambient oxygen conditions so the hemoglobin was equilibrated with air oxygen. One group of control units (Ambient Pouched) was sealed in foil pouches and the other (Ambient Not Pouched) was not sealed in a foil pouch. All units were stored in a 25°C incubator.

At intervals, duplicate packages were pulled from each group and samples were taken from each flexible container with syringe and needle. Aliquots of the 10 g/dl samples were filtered, diluted 50X in phosphate buffer and spectrophotometrically analyzed for methemoglobin content.

After an initial three day oxidation phase, spontaneous autoreduction of methemoglobin was evident in the deoxygenated test group. Over the next sixty days the methemoglobin fell 17.7% in the deoxygenated solution while the oxygenated control group solutions continued to oxidize unabated to over ninety percent methemoglobin (Table 5; FIG. 5). Although the experiment was terminated after 63 days, it would be anticipated that the 15% methemoglobin level preferred for an oxygen-carrying solution would have been reached.

TABLE 4

| Changes in Percent Methemoglobin Content in 10 g/dl Solutions of Diaspirin Cross-Linked Hemoglobin in Lactated Ringer's Packaged In Flexible Containers | | | |
|---|---|---|---|
| TIME/DAYS | PERCENT METHEMOGLOBIN | | |
| | DEOXYGENATED TEST GROUP | OXYGENATED CONTROL GROUPS | |
| | | Pouched | Not Pouched |
| PRE* | 8.9 | 8.9 | 8.9 |
| POST** | 15.5 | 8.9 | 8.9 |
| 1 | 36.6 | 19.9 | 18.4 |
| 3 | 43.9 | 38.4 | 36.9 |
| 5 | 42.5 | 50.3 | 48.3 |
| 7 | 41.4 | 57.6 | 56.1 |
| 14 | 37.2 | 73.5 | 71.7 |
| 28 | 32.2 | 86.4 | 94.3 |
| 63 | 26.2 | 94.3 | 95.3 |
| PRE* = preprocessing; the level of methemoglobin before deoxygenation and packaging. POST** = post-processing; the level of methemoglobin after deoxygenation and packaging were completed. | | | |

Example 5

Plastic bags were filled with 50 ml of solution and sealed in foil pouches. The packaging operation was carried out in an isolator continuously purged with ultra-pure nitrogen.

The isolator technology described in Example 4 was again used in this experiment. In this example, however, the isolator was fogged with $H_2O_2$ after sealing to sterilize all components used in the packaging operation and are rigorous efforts were employed to purge oxygen from the isolator, the packing containers and the hemoglobin solution. After fogging, the isolator was purged with low-$O_2$ grade nitrogen. The purge line was then switched to ultra-pure nitrogen with an in-line oxygen trap and the isolator was purged further. All components were carefully purged with the same ultra-pure nitrogen. Additionally, Ageless (Mitsubishi Chemical Co., Inc.) oxygen absorbing packets were added to certain outer envelopes to further reduce oxygen in the container.

The control group did not receive oxygen absorbing packets. They were stored at 25°C. One or four packets were added to the outer envelopes of the two 25°C test groups. One packet per container was included in another test group that was stored at 5°C.

In contrast to the results observed in Example 4 almost no increase in methemoglobin was observed over the first three days in samples from any of the test or control groups in this experiment. This result indicated that the initial solution oxygen level was sufficiently low to only allow a rate of autoxidation essentially equal to the rate of autoreduction (Table 6; FIG. 6).

Autoreduction was evident on day three in the units from the 25°C test group containing four oxygen absorbing packets. Methemoglobin could not be detected, after 32 or 56 days, in any units from the 25°C test group containing one Ageless (Mitsubishi Chemical Co.) packet or the control group. Hemoglobin oxidation continued to day five in the 5°C stored units and remained at the five day level for the remainder of the study (56 days).

Duplicate samples from each test and control group were submitted for sterility testing and were found to be negative in aerobic and anaerobic culture and did not exceed minimum acceptable pyrogen levels.

**TABLE 5**

**Changes in Percent Methemoglobin Content In 10 g/dl Solutions of Diaspirin Cross-Linked Hemoglobin in Lactated Ringer's Packaged In Flexible Containers**

| TIME/ | Temp: | PERCENT METHEMOGLOBIN 25°C | 25°C | 25°C | 5°C |
|---|---|---|---|---|---|
| DAYS | # Packets: | 0 | 1 | 4 | 1 |
| PRE* | | 4.5 | 4.5 | 4.5 | 4.5 |
| POST** | | 5.5 | 5.5 | 5.5 | 5.5 |
| 1 | | 5.9 | 5.6 | 5.7 | 5.7 |
| 3 | | 5.6 | 5.5 | 5.4 | 6.0 |
| 5 | | 5.4 | 5.0 | 4.8 | 5.8 |
| 7 | | 4.5 | 4.3 | 3.9 | 6.0 |
| 13 | | 3.9 | 3.1 | NS | 5.7 |
| 32 | | 0 | 0 | NS | 5.6 |
| 56 | | 0 | 0 | NS | 6.3 |

PRE* = preprocessing; the level of methemoglobin before deoxygenation and packaging.

POST** = post-processing; the level of methemoglobin after deoxygenation and packaging were completed.

NS = not sampled.

Example 6

The purpose of this experiment was to study the temperature dependence of the hemoglobin oxidation and reduction reactions that take place during storage in the system. Again, a 10 g/dl solution of diaspirin cross-linked hemoglobin in lactated Ringer's was packaged in flexible containers and foil pouches using the isolator technology described in Example 4 and the more rigorous steps to exclude oxygen described in Example 5.

Four groups of test units were packaged, each unit containing one 200 ml capacity oxygen-absorbing packet. The test groups were stored at 5°C, 25°C, 30°C and 45°C respectively. Control group units were stored at 25°C without Ageless (Mitsubishi Chemical Co., Inc.) packets. Duplicate samples from each group were submitted for sterility and pyrogen testing.

The data from this experiment are given in Table 6 and FIG. 6. Results at 25°C were similar to those obtained in Examples 4 and 5. The 45°C response was particularly notable since the oxidative deoxygenation phase required less than a day to complete before the onset of autoreduction. All samples submitted were found to be sterile in aerobic and anaerobic culture and non-pyrogenic

TABLE 6

Changes in Percent Methemoglobin Content in a Solution of 10g/dl
Diaspirin Cross-Linked Hemoglobin in Lactated Ringer's Packaged
in Flexible Containers

| | | PERCENT METHEMOGLOBIN | | | | |
|---|---|---|---|---|---|---|
| TIME/ | Temp: | 25°C | 5°C | 25°C | 30°C | 45°C |
| DAYS | # Packets: | 0 | 1 | 1 | 1 | 1 |
| PRE* | | 5.0 | 5.0 | 5.0 | 5.0 | 5.0 |
| POST** | | 7.7 | 7.7 | 7.7 | 7.7 | 7.7 |
| 1 | | 9.7 | 9.2 | 9.2 | 9.0 | 5.9 |
| 3 | | 8.7 | 8.9 | 8.4 | 8.1 | 1.6 |
| 7 | | 8.8 | 9.3 | 7.0 | 5.9 | ND |
| 14 | | 7.7 | 9.6 | 5.0 | 3.2 | 2.3 |
| 31 | | 2.6 | 8.6 | 1.0 | 0 | ND |
| 42 | | 0.1 | 7.6 | ND | NS | NS |
| 59*** | | 1.0 | 8.6 | 0.6 | NS | NS |
| 175 | | 2.0 | NS | 1.2 | NS | NS |
| 187 | | NS | 5.8 | NS | NS | NS |
| 231 | | 0.6 | NS | 0.6 | NS | NS |

PRE* = preprocessing; the level of methemoglobin before
deoxygenation and packaging.

POST** = post-processing; the level of methemoglobin after
deoxygenation and packaging were completed.

*** = measurements to determine hemoglobin moieties collected
on a Cary 2200 from day 59 to the end of study.
Previous measurements were from a Hewlett-Packard 8451A
instrument.

ND = not detected.

NS = not sampled.

Example 7

In this experiment we evaluated whether inclusion of oxygen absorbing packets in overpouches may be beneficial over a long storage interval. The test groups in this experiment contained one, two or four type S-200 Ageless (Mitsubishi Chemical Co.) packets in the package head space corresponding 200, 400 or 800 ml of oxygen absorbing capacity, respectively. Once again, a 10 g/dl solution of diaspirin cross-linked hemoglobin in lactated Ringer's was packaged in flexible containers and foil pouches using the isolator technology described in Example 4 and the more rigorous steps to exclude oxygen described in Example 5. The study was designed for a one year duration.

There may have been a small effect on the rate of autoreduction comparing control group sample with samples containing oxygen absorbing packets, but essentially all groups displays virtually identical autoxidation-autoreduction behavior (Table 7; FIG. 8).

12

TABLE 7

Changes in Percent Methemoglobin Content In a Solution of
10 g/dl Diaspirin Cross-Linked Hemoglobin in Lactated Ringer's
Packaged in Flexible Containers

| | | PERCENT METHEMOGLOBIN | | | |
|---|---|---|---|---|---|
| TIME/ | Temp: | 25°C | 25°C | 25°C | 25°C |
| DAYS | # Packets: | 0 | 1 | 2 | 4 |
| PRE* | | 5.4 | 5.4 | 5.4 | 5.4 |
| POST** | | 10.8 | 10.8 | 10.8 | 10.8 |
| 3 | | 11.6 | 11.0 | 11.3 | 12.4 |
| 5 | | 11.2 | 10.6 | 11.1 | 11.0 |
| 7 | | 10.1 | 8.1 | 9.6 | 9.2 |
| 20 | | 6.2 | 4.5 | 4.4 | 4.7 |
| 56 | | 1.9 | 1.5 | 1.9 | 1.6 |
| 112 | | 2.5 | 2.1 | 2.2 | 1.5 |
| 168 | | 1.5 | 1.2 | 1.8 | 1.2 |

PRE* = preprocessing; the level of methemoglobin before
deoxygenation and packaging.

POST** = post-processing; the level of methemoglobin after
deoxygenation and packaging were completed.

Samples from examples 6 and 7 were assayed for enzymes known to protect hemoglobin against oxidative damage. See Table 8.

TABLE 8

| Activities[a] of Reductive Enzymes per Gram of Hemoglobin | | | | |
|---|---|---|---|---|
| TYPE OF HEMOGLOBIN | HEMOGLOBIN (g/100 mL) | GLUTATHIONE REDUCTASE | | METHEMOGLOBIN REDUCTASE |
| | | No FAD | With FAD | |
| Human Erythrocytes | | 7.18 | 10.4 | 19.21 |
| Human Hemolysate | 1.03 | | | 13.61 |
| DeoxyDCLHb | 9.97 | 0.35 | 0.56 | Not Detected |
| DeoxyDCLHb | 9.72 | 1.58 | 0.04 | Not Detected |

[a] Activity is defined on the basis that one unit of enzyme catalyzes the conversion of 1 $\mu$mole of substrate per minute. In ancillary studies of parallel samples of diaspirin cross-linked hemoglobin, it has been shown that NADH-methemoglobin reductase, catalase, and superoxide dismutase activities could not be detected.

The absence of normal reductive enzymatic activity in these sample confirms that autoreduction of hemoglobin under the conditions described in this Invention is independent of enzyme systems present in unpurified samples.

**Claims**

1. A method of preparing a hemoglobin based composition having methemoglobin in an amount not greater than 15% of total hemoglobin, wherein the composition may function as an oxygen carrying solution upon administration to a patient,

the method comprising removing oxygen from an oxygen impermeable container, adding a purified substantially deoxygenated hemoglobin solution to the container and storing the container at a temperature of 5°C to 45°C for a sufficient time to permit autoreduction of methemoglobin.

2. A method of reducing methemoglobin in a solution of substantially deoxygenated and purified methemoglobin, the method comprising removing oxygen from an oxygen impermeable container, adding the deoxygenated and purified hemoglobin solution to the container, and storing the container at a temperature of 5°C to 45°C for a sufficient time for autoreduction of methemoglobin in the solution to occur, such that the resultant solution contains methemoglobin in an amount not greater than 15% of total hemoglobin, so that the solution may function as an oxygen carrying solution upon administration to a patient.

3. The method of Claim 1 or 2, wherein said hemoglobin composition is stored at between -80°C and 45°C, after said autoreduction has been effected.

4. A method of storing hemoglobin for use as an oxygen carrying solution upon administration to a patient, the method comprising
   purging an oxygen impermeable container with nitrogen,
   deoxygenating a purified hemoglobin solution,
   filling the oxygen-purged impermeable container with an aliquot of the deoxygenated, purified hemoglobin solution,
   sealing the container,
   storing the container for a sufficient time to permit scavenging of residual oxygen from the hemoglobin solution,
   storing the container further at a temperature of 5°C to 45°C to permit autoreduction of methemoglobin to a level not greater than 15% of total hemoglobin, and
   further storing the hemoglobin solution between -270°C and 45°C.

5. A method according to any preceding claim, wherein the container is sealed in an isolator, the isolator is rigorously purged of oxygen using nitrogen and the container is filled with an aliquot of the deoxygenated hemoglobin solution.

6. A method according to any preceding claim, wherein the container comprises a primary container and an outer secondary container of very low oxygen permeability.

7. A method for packaging a hemoglobin solution, comprising purging an oxygen impermeable container with nitrogen,
   preparing a thoroughly deoxygenated, purified hemoglobin solution,
   filling said purged container with an aliquot of said thoroughly deoxygenated, purified hemoglobin solution, and
   sealing the container,
   whereby methemoglobin in the solution is autoreducible to a level not greater than 15% of the total hemoglobin when the sealed container is stored at a temperature of 5°C to 45°C for a sufficient amount of time to permit autoreduction to said level such that the solution is usable as an oxygen carrying solution upon administration to a patient.

**Patentansprüche**

1. Verfahren zum Herstellen einer Zusammensetzung auf Hämoglobinbasis, die Methämoglobin in einer Menge von nicht mehr als 15 % Gesamthämoglobin hat, wobei die Zusammensetzung bei Verabreichung an einen Patienten als eine sauerstofftragende Lösung wirken kann,
   wobei das Verfahren die folgenden Schritte aufweist: Entfernen von Sauerstoff aus einem für Sauerstoff undurchlässigen Behälter, Zufügen einer gereinigten, im wesentlichen desoxygenierten Hämoglobinlösung zu dem Behälter und Lagern des Behälters bei einer Temperatur von 5 °C bis 45 °C für eine ausreichende Zeitdauer, um die Autoreduktion von Methämoglobin zu gestatten.

2. Verfahren zur Reduktion von Methämoglobin in einer Lösung aus im wesentlichen desoxygeniertem und gereinigtem Methämoglobin, wobei das Verfahren die folgenden Schritte aufweist: Entfernen von Sauerstoff aus einem für Sauerstoff undurchlässigen Behälter, Zufügen der desoxygenierten und gereinigten Hämoglobinlösung zu dem Behälter und Lagern des Behälters bei einer Temperatur von 5 °C bis 45 °C für eine ausreichende Zeitdauer, damit die Autoreduktion von Methämoglobin in der Lösung stattfindet, derart daß die resultierende Lösung Methämoglobin in einer Menge von nicht mehr als 15 % Gesamthämoglobin enthält, so daß die Lösung bei Verabreichung an einen Patienten als eine sauerstofftragende Lösung wirken kann.

3. Verfahren nach Anspruch 1 oder 2, wobei die Hämoglobinzusammensetzung nach Durchführung der Autoreduktion bei einer Temperatur zwischen -80 °C und 45 °C gelagert wird.

4. Verfahren zum Lagern von Hämoglobin zur Verwendung als eine sauerstofftragende Lösung bei Verabreichung an einen Patienten, wobei das Verfahren die folgenden Schritte aufweist:
    Spülen eines für Sauerstoff undurchlässigen Behälters mit Stickstoff,
    Desoxygenieren einer gereinigten Hämoglobinlösung,
    Füllen des gespülten für Sauerstoff undurchlässigen Behälters mit einem Aliquot der desoxygenierten, gereinigten Hämoglobinlösung,
    hermetisches Verschließen des Behälters,
    Lagern des Behälters für eine ausreichende Zeitdauer, um das Entfernen von Restsauerstoff aus der Hämoglobinlösung zu gestatten,
    weiteres Lagern des Behälters bei einer Temperatur von 5 °C bis 45 °C, um die Autoreduktion von Methämoglobin auf einen Wert von nicht mehr als 15 % Gesamthämoglobin zu gestatten, und
    weiteres Lagern der Hämoglobinlösung bei einer Temperatur zwischen -270 °C und 45 °C.

5. Verfahren nach einem der vorhergehenden Ansprüche, wobei der Behälter in einem Isolator hermetisch eingeschlossen wird, der Isolator unter Einsatz von Stickstoff rigoros von Sauerstoff gereinigt wird und der Behälter mit einem Aliquot der desoxygenierten Hämoglobinlösung gefüllt wird.

6. Verfahren nach einem der vorhergehenden Ansprüche, wobei der Behälter einen Primärbehälter und einen äußeren Sekundärbehälter sehr geringer Sauerstoffdurchlässigkeit aufweist.

7. Verfahren zum Verpacken einer Hämoglobinlösung, das die folgenden Schritte aufweist: Spülen eines für Sauerstoff undurchlässigen Behälters mit Stickstoff,
    Herstellen einer gründlich desoxygenierten, gereinigten Hämoglobinlösung,
    Füllen des gespülten Behälters mit einem Aliquot der gründlich desoxygenierten, gereinigten Hämoglobinlösung und
    hermetisches Verschließen des Behälters,
    wobei Methämoglobin in der Lösung auf einen Wert von nicht mehr als 15 % Gesamthämoglobin autoreduzierbar ist, wenn der hermetisch verschlossene Behälter bei einer Temperatur von 5 °C bis 45 °C für eine ausreichende Zeitdauer gelagert wird, um die Autoreduktion auf diesen Wert zu gestatten, so daß die Lösung bei Verabreichung an einen Patienten als eine sauerstofftragende Lösung verwendbar ist.

**Revendications**

1. Procédé de préparation d'une composition à base d'hémoglobine contenant de la méthémoglobine en une quantité non supérieure à 15% de l'hémoglobine totale, dans lequel la composition peut fonctionner comme une solution de transport d'oxygène lors de l'administration à un patient,
le procédé comprenant l'élimination de l'oxygène d'un récipient imperméable à l'oxygène, l'addition d'une solution d'hémoglobine purifiée sensiblement désoxygénée au récipient, et le stockage du récipient à une température de 5°C à 45°C pendant un temps suffisant pour permettre l'autoréduction de la méthémoglobine.

2. Procédé de réduction de la méthémoglobine dans une solution de méthémoglobine sensiblement désoxygénée et purifiée,
le procédé comprenant l'élimination de l'oxygène d'un récipient imperméable à l'oxygène, l'addition de la solution d'hémoglobine désoxygénée et purifiée au récipient, et le stockage du récipient à une température de 5°C à 45°C pendant un temps suffisant pour que l'autoréduction de la méthémoglobine dans la solution ait lieu, de sorte que la solution résultante contient de la méthémoglobine en une quantité non supérieure à 15% de l'hémoglobine totale et que la solution peut fonctionner comme une solution de transport d'oxygène lors de l'administration à un patient.

3. Procédé suivant la revendication 1 ou 2, dans lequel ladite composition d'hémoglobine est stockée à une température comprise entre -80°C et 45°C après achèvement de ladite autoréduction.

4. Procédé de stockage d'hémoglobine utilisable comme solution de transport d'oxygène lors de l'administration à un patient,
le procédé comprenant :
le balayage d'un récipient imperméable à l'oxygène avec de l'azote,
la désoxygénation d'une solution d'hémoglobine purifiée,
le remplissage du récipient imperméable purgé de l'oxygène, avec une partie aliquote de la solution d'hémoglobine purifiée désoxygénée,
la fermeture étanche du récipient,
le stockage du récipient pendant un temps suffisant pour permettre l'élimination de l'oxygène résiduel de la solution d'hémoglobine,
le stockage du récipient en outre à une température de 5°C à 45°C pour permettre l'autoréduction de la méthémoglobine à un niveau non supérieur à 15% de l'hémoglobine totale, et
la poursuite du stockage de la solution d'hémoglobine entre -270°C et 45°C.

5. Procédé suivant une quelconque des revendications précédentes, dans lequel le récipient est hermétiquement placé dans un isolateur, l'isolateur est rigoureusement purgé de l'oxygène au moyen d'azote, et le récipient est rempli avec une partie aliquote de la solution d'hémoglobine désoxygénée.

6. Procédé suivant une quelconque des revendications précédentes, dans lequel le récipient comprend un récipient primaire et un récipient secondaire extérieur de très faible perméabilité à l'oxygène.

7. Procédé pour l'emballage d'une solution d'hémoglobine, comprenant :
le balayage d'un récipient imperméable à l'oxygène avec de l'azote,
la préparation d'une solution d'hémoglobine purifiée, complètement désoxygénée,
le remplissage dudit récipient balayé, avec une partis aliquote de ladite solution d'hémoglobine purifiés complètement désoxygénée, et
la fermeture étanche du récipient, de sorte que la méthémoglobine dans la solution est autoréductible à une valeur non supérieure à 15% de l'hémoglobine totale lorsque le récipient fermé est stocké à une température de 5°C à 45°C pendant un temps suffisant pour permettre l'autoréduction à cette valeur, de sorte que la solution est utilisable comme solution de transport d'oxygène lors de l'administration à un patient.

Fig. 1

CHANGES IN CONCENTRATION OF HEMOGLOBIN MOIETIES VERSUS TIME IN A SOLUTION OF DIASPIRIN ALPHA-ALPHA CROSS-LINKED HEMOGLOBIN IN LACTATED RINGER'S SEALED IN SCREW-CAPPED CUVETTES AT 25°C

MICROMOLAR CONCENTRATION

STORAGE TIME (DAYS)

o oxy-hemoglobin
△ deoxy-hemoglobin
□ methemoglobin

EP 0 495 047 B1

CHANGES IN MEAN PERCENT OF DIFFERENT HEMOGLOBIN MOIETIES VERSUS
TIME IN A DIASPIRIN ALPHA-ALPHA CROSS-LINKED HEMOGLOBIN
COMPOSITION IN RINGER'S LACTATE SOLUTION STORED AT 25°C
IN SCREW-CAPPED CUVETTES

Fig. 2

PERCENT DIASPIRIN ALPHA-ALPHA CROSS-LINKED
HEMOGLOBIN COMPONENTS ( MEAN + S.D. )

STORAGE TIME (DAYS)

△ METHEMOGLOBIN
□ OXY-HEMOGLOBIN
○ DEOXY-HEMOGLOBIN

CHANGES IN CONCENTRATION OF DIFFERENT HEMOGLOBIN
MOIETIES VERSUS TIME IN A DIASPIRIN ALPHA-ALPHA
CROSS-LINKED HEMOGLOBIN COMPOSITION IN PHOSPHATE BUFFER
(pH 7.40) STORED AT 25°C IN SCREW-CAPPED CUVETTES

o METHEMOGLOBIN
□ OXY-HEMOGLOBIN
● DEOXY-HEMOGLOBIN
△ TOTAL HEMOGLOBIN

STORAGE TIME (DAYS)

MICROMOLAR CONCENTRATION

Fig. 3

CHANGES IN METHEMOGLOBIN CONTENT VERSUS TIME
IN 10 g/dl DIASPIRIN ALPHA-ALPHA CROSS-LINKED
HEMOGLOBIN STORED AT 25°C IN FLEXIBLE CONTAINERS

Fig. 4

Fig. 5

CHANGES IN METHEMOGLOBIN CONTENT VERSUS TIME IN 10 g/dl DIASPIRIN ALPHA-ALPHA CROSS-LINKED HEMOGLOBIN STORED IN FLEXIBLE CONTAINERS

■ 25°C, 1 PACK
□ 25°C, 4 PACKS
◆ 5°C, 1 PACK
◇ 25°C, NO PACKS

PERCENT METHEMOGLOBIN

TIME (DAYS)

CHANGES IN METHEMOGLOBIN CONTENT VERSUS TIME IN 10 g/dl DIASPIRIN
ALPHA-ALPHA CROSS-LINKED HEMOGLOBIN STORED IN FLEXIBLE CONTAINERS

Fig. 6

CHANGES IN METHEMOGLOBIN CONTENT VERSUS TIME IN 10 g/dl DIASPIRIN ALPHA-ALPHA CROSS-LINKED HEMOGLOBIN STORED IN FLEXIBLE CONTAINERS

FIG. 7